Europäisches Patentamt

European Patent Office

Office européen des brevets

(19)

(11) **EP 1 185 220 B1**

(12) **EUROPÄISCHE PATENTSCHRIFT**

(45) Veröffentlichungstag und Bekanntmachung des Hinweises auf die Patenterteilung:
**07.04.2004 Patentblatt 2004/15**

(51) Int Cl.7: **A61F 2/16**

(21) Anmeldenummer: **00935140.4**

(22) Anmeldetag: **29.05.2000**

(86) Internationale Anmeldenummer:
**PCT/EP2000/004888**

(87) Internationale Veröffentlichungsnummer:
**WO 2000/076426 (21.12.2000 Gazette 2000/51)**

(54) **INTRAOKULARLINSE**

INTRAOCULAR LENS

LENTILLE INTRA-OCULAIRE

(84) Benannte Vertragsstaaten:
**AT BE CH CY DE DK ES FI FR GB GR IE IT LI LU MC NL PT SE**

(30) Priorität: **10.06.1999 DE 19926512**

(43) Veröffentlichungstag der Anmeldung:
**13.03.2002 Patentblatt 2002/11**

(73) Patentinhaber: **Acritec GmbH**
**16548 Glienicke (DE)**

(72) Erfinder:
• **STORK, Wilhelm**
**D-76831 Impflingen (DE)**

• **KREINER, Christine, F.**
**D-81545 München (DE)**

(74) Vertreter: **Nöth, Heinz**
**Patent Attorney,**
**Arnulfstrasse 25**
**80335 München (DE)**

(56) Entgegenhaltungen:
**EP-A- 0 180 887       EP-A- 0 276 331**
**EP-A- 0 342 895       EP-A- 0 367 878**
**EP-A- 0 458 508**

**Beschreibung**

Die Erfindung betrifft eine Intraokularlinse nach dem Oberbegriff des Patentanspruches 1.

Aus EP-A-0 458 508 ist eine derartige Intraokularlinse bekannt, bei welcher um die Linsenachse konzentrische ringförmige Zonen vorgesehen sind, bei denen der Weglängenunterschied des Strahlengangs zwischen benachbarten Zonen ein ganzzahliges Vielfaches von n 2 der Designwellenlänge ist. Die ringförmigen Zonen haben eine prismatische Mikrostruktur, welche in der Brennebene zu einer um den Brennpunkt angeordneten zusätzlichen ringförmigen Lichtverteilung führt. Hieraus resultiert eine verringerte Qualität des auf der Netzhaut erzeugten Bildes.

Die aus EP 0 537 643 B1 bekannte Intraokularlinse kann als monofokale Linse dadurch relativ dünn ausgebildet werden, daß die Brechkraft aus einem refraktiven und einem diffraktiven Anteil zusammengesetzt ist. Der am Auge anzubringende Schnitt bei der Implantation kann klein gehalten werden. Aus dem diffraktiven Feinstrukturanteil resultierende Lichtsteuungen können die Qualität des auf der Netzhaut erzeugten Bildes beeinflussen.

Aufgabe der Erfindung ist es, eine Intraokularlinse der eingangs genannten Art zu schaffen, bei welcher mit geringer Linsendicke auf der Netzhaut ein Bild mit verbesserter Qualität erzeugt wird.

Diese Aufgabe wird erfindungsgemäß durch die kennzeichnenden Merkmale des Patentanspruches 1 gelöst.

Bei der erfindungsgemäßen Intraokularlinse ist um einen zentralen Linsenbereich, der insbesondere refraktive Eigenschaften hat, wenigstens ein ringförmiger Linsenbereich angeordnet, der mit dem zentralen Linsenbereich einen gemeinsamen Fokus bildet, wobei in dem ringförmigen Linsenbereich konzentrische um die optische Linsenachse angeordnete ringförmige Zonen vorgesehen sind, bei denen der Weglängenunterschied des Strahlenganges zwischen benachbarten Zonen ein ganzzahliges Vielfaches der Designwellenlänge ist.

In bevorzugter Weise ist die Designwellenlänge im grünen Spektralbereich des sichtbaren Lichtes im Bereich von beispielsweise 550 nm vorgesehen.

Der Weglängenunterschied der benachbarten Zonen kann durch den Brechungsindex bzw. durch entsprechende Materialwahl und/oder die Geometrie der jeweiligen Zone eingestellt werden.

Die Krümmung des meridialen Schnittes des optischen Linsenteils ist asphärisch ausgebildet, wobei die Zonen mit den Weglängenunterschieden in dem Randbereich vorgesehen sind, in welchem sich die Abweichung des asphärischen Verlaufs von der sphärischen Kurve auswirkt.

Diese ringförmigen Zonen, welche konzentrisch um die optische Linsenachse angeordnet sind, sind insbesondere sägezahnförmig ausgebildet. Diese Zonen besitzen zur Bildung einer monofokalen Intraokularlinse die gleiche Brechkraft wie der zentrale, insbesondere refraktive Linsenbereich. Beide Teile tragen zu einem scharfen Bild, das auf der Netzhaut des Auges erzeugt wird, bei.

Zur Bildung einer bifokalen Linse kann der optische Linsenteil mit einer zusätzlichen diffraktiven Feinstruktur ausgestattet sein, die sich über den gesamten optischen Linsenteil erstrecken kann oder in bevorzugter Weise nur am zentralen den refraktiven Anteil bildenden Linsenbereich vorgesehen ist. In aller Regel reicht dies aus, da die bifokale Funktion nur bei einer dem Tageslicht entsprechenden Helligkeit erforderlich ist und die Pupillenöffnung des Auges im wesentlichen nur im Bereich des zentralen, den refraktiven Anteil enthaltenden Linsenbereiches geöffnet ist. Die zusätzliche diffraktive Feinstruktur insbesondere in Form von um die optische Linsenachse angeordneten konzentrischen Zonen kann so ausgebildet sein, daß benachbarte Zonen einen Weglängenunterschied des Strahlenganges erzeugen, der ein Bruchteil der Designwellenlänge, z.B. 0,4 oder 0,6 beträgt.

Anhand der Figuren wird an Ausführungsbeispielen die Erfindung noch näher erläuter. Es zeigt:

Figur 1    eine schnittbildliche Darstellung durch eine Hälfte eines Linsenkörpers einer Intraokularlinse, und

Figur 2    eine graphische Darstellung zur Erläuterung einer zusätzlichen diffraktiven Feinstruktur, zur Bildung einer bifokalen Intraokularlinse;

Der in den Figuren dargestellte optische Linsenteil 1 einer Intraokularlinse besitzt einen zentralen insbesondere refraktiven Linsenbereich 2 und einen ringförmig um den zentralen Linsenbereich 2 angeordneten Linsenbereich 3. Der ringförmige Linsenbereich 3 befindet sich in einer Randzone des Linsenkörpers. Beim dargestellten Ausführungsbeispiel sind sowohl auf der Vorderseite als auch auf der Rückseite des Linsenkörpers Feinstrukturelemente, insbesondere mit Sägezahnform in konzentrischen Zonen um die optische Achse 6 des Linsenteils 1 angeordnet. Es ist jedoch auch möglich, die sägezahnartigen Zonen nur auf einer Linsenseite (Vorderseite oder Rückseite) vorzusehen.

Benachbarte Zonen besitzen einen Weglängenunterschied des jeweiligen Strahlengangs, der einem ganzzahligen Vielfachen von zwei oder mehr der Designwellenlänge entspricht. Durch unterschiedliche Auswahl des Materials in den jeweiligen benachbarten ringförmigen Zonen und der damit verbundenen unterschiedlichen Brechungsindizes und/oder der Geometrie, insbesondere der Sägezahnform kann dieser Weglängenunterschied der jeweiligen Strahlengänge erreicht werden.

Ein äußerer umlaufender Rand 4 des Linsenkörpers besitzt einen etwa halbkreisförmigen Querschnitt, mit einem Radius von 0,165 mm. Der halbkreisförmige Rand beginnt bei einem radialen Abstand von etwa 2,835 mm von der optischen Achse 6. Zwischen dem Rand 4 und dem ringförmigen Linsenbereich 3 mit den sägezahnartigen Zonen kann ein ebenes Geradenstück 5 vorgesehen sein. Dies ist insbesondere dann der Fall, wenn die äußerste Sägezahnzone vor dem halbkreisförmigen Linsenrand 4 nicht mehr vollständig ausgeführt werden kann. Der Durchmesser der Linse beträgt ca. 6 mm. In bevorzugter Weise sind wenigstens drei ringförmige Sägezahnzonen im ringförmigen Linsenbereich 3 in der Nähe des Linsenrandes 4 vorgesehen.

Die verschiedenen Kurvenabschnitte werden durch verschiedene Funktionen in ihren jeweiligen Abschnitten beschrieben.

Der optische Linsenteil wird durch die nachfolgende Funktion beschrieben:

$$z_{Asph}(r) = R - \sqrt{R^2 - r^2} + a_4 \cdot r^4 + a_6 \cdot r^6 + a_8 \cdot r^8 + a_{10} \cdot r^{10} + \ldots.$$

wenn $r < r_{fres\_begin}$ Der ringförmige Linsenbereich 3 wird durch die Floorfunktion beschrieben:

$$z\_fres(r) = z_{Asph}(r) - Floor\left[\frac{z_{Asph}(r) - z_{Asph}(r_{fres\_begin})}{Zahntiefe} + 1\right] \cdot Zahntiefe$$

wenn $r_{fres\_begin} < r < r_{fres\_end}$

Das Geradenstück 5 wird durch die Gerade beschrieben:

$$z(r) = z_{Asph}(r_{fres\_begin})$$

wenn $r_{fres\_end} < r < r_{Kreis\_begin}$

Der Randbereich wird durch eine Kreisfunktion mit dem Radius R = 0.165 mm beschrieben:

$$z_{Kreis} = z_{Mpunkt} - \sqrt{R^2 - (r - x_{Mpunkt})^2}$$

wenn $r_{Kreis\_begin} < r < r_{max}$

mit $z_{Mpunkt}$ = z-Koordinate des Mittelpunkts des Randkreises, $x_{Mpunkt}$ = r-Koordinate des Mittelpunkts des Randkreises. $r_{max}$ ist der maximale Abstand von der Achse bzw. der halbe Durchmesser. Die r-Koordinaten des Randkreises sind bis auf $r_{fres\_begin}$ bei allen Formeinsätzen gleich.

In bevorzugter Weise befinden sich die Zonen mit den Weglängenunterschieden des Linsenbereiches 3 im Bereich der Abweichung der Asphäre von der sphärischen Kurve. Der refraktive Anteil wird vom zentralen Linsenbereich 2 gebildet, welcher in bevorzugter Weise die sphärische Linsenform aufweist.

Zur Bildung einer monofokalen Linse sind der zentrale Linsenbereich 2 und der ringförmige Linsenbereich 3 so gestaltet, dass sie exakt den gleichen Fokus besitzen und in allen Zonen des optischen Linsenteils 1 ein gemeinsames Bild erzeugt wird. Die optischen Weglängenunterschiede der Strahlengänge in benachbarten Zonen sind dabei genau auf ein ganzzahliges Vielfaches einer mittleren Wellenlänge des sichtbaren Spektrums, insbesondere auf etwa 550mm (Designwellenlänge) angepaßt. Die Linse liefert daher bis in den Randbereich ein perfektes Bild. Die Tiefe der konzentrischen Sägezahnzonen verringert sich hierbei von Zone zu Zone um 0,3 µm.

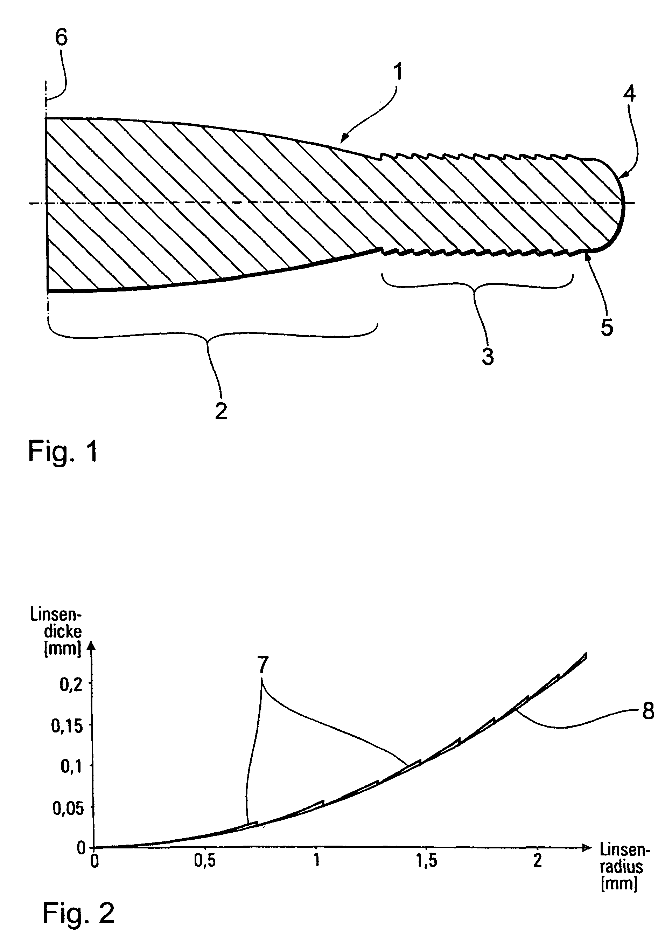

Zur Bildung einer bifokalen Linse wird am optischen Linsenteil 1 eine zusätzliche diffraktive Feinstruktur vorgesehen. Diese Feinstruktur ist bevorzugt als diffraktives Fresnelmuster ausbildet und besitzt die Form ringförmiger Feinstrukturelemente 7 in Sägezahnform (Figur 2). Die Figur 2 zeigt den im wesentlichen sphärischen Verlauf der Schnittkurve des zentralen den refraktiven Anteil 2 bildenden Linsenbereiches an einer Seite. Ausgehend von einer refraktiven Grundkurve 8 mit im wesentlichen sphärischen Schnittkurvenverlauf besitzen die diffraktiven ringförmigen Sägezahnzonen Zahntiefen von 1,5µm bis 2,8µm. Der Weglängenunterschied zwischen benachbarten Zonen kann ein Bruchteil, z.B. 0,4 oder 0,6 der Designwellenlänge betragen. Das zusätzliche diffraktive Feinstrukturmuster ist bevorzugt im zentralen den refraktiven Anteil bildenden Linsenbereich vorgesehen. Es kann sich jedoch auch über den ringförmigen Linsenbereich 3 erstrecken und die in diesem Bereich befindlichen Zonen überlagern. Wie die Figur 2 zeigt, sind die zusätzlichen diffraktiven Feinstrukturelemente 7 ausgehend von der refraktiven Grundkurve 8 in die Oberfläche des Linsenkörpers, insbesondere im zentralen Bereich eingeformt.

## EP 1 185 220 B1

**[Bezugszeichenliste]**

1 optischer Linsenteil
2 zentraler Linsenbereich
3 ringförmiger Linsenbereich
4 umlaufender Rand
5 Geradenstück
6 optische Achse
7 zusätzliche diffraktive Feinstrukturelemente
8 refraktive Grundkurve

**Patentansprüche**

1. Intraokularlinse mit einem optischen Linsenteil, der einen zentralen Linsenbereich (2) und wenigstens einen weiteren diesen umgebenden ringförmigen Linsenbereich (3) aufweist, wobei der zentrale Linsenbereich (2) und der wenigstens eine ringförmige Linsenbereich (3) einen gemeinsamen Fokus bilden, und wobei der ringförmige Linsenbereich (3) konzentrische ringförmige Zonen aufweist, bei denen der Weglängenunterschied des Strahlenganges zwischen benachbarten Zonen ein ganzzahliges Vielfaches von n ≥ 2 der Designwellenlänge ist, **dadurch gekennzeichnet, dass** der optische Linsenteil im Meridianschnitt einen asphärischen Krümmungsverlauf hat und dass der ringförmige Bereich (3) mit den die unterschiedlichen Weglängen aufweisenden konzentrischen Zonen in dem Linsenteil angeordnet ist, in welchem sich der asphärische Krümmungsverlauf auswirkt.

2. Intraokularlinse nach Anspruch 1, **dadurch gekennzeichnet, daß** der Weglängenunterschied durch den Brechungsindex bzw. das Material und/oder die Geometrie der jeweiligen Zone eingestellt ist.

3. Intraokularlinse nach Anspruch 1 oder 2, **dadurch gekennzeichnet, daß** die ringförmigen Zonen sägezahnartig ausgebildet sind.

4. Intraokularlinse nach einem der Ansprüche 1 bis 3, **dadurch gekennzeichnet, daß** die ringförmigen Zonen auf der Vorder- und/oder Rückseite des Linsenkörpers (1) vorgesehen sind.

5. Intraokularlinse nach einem der Ansprüche 1 bis 4, **dadurch gekennzeichnet, daß** im zentralen Linsenbereich (2) ein refraktiver Anteil (2) gebildet ist.

6. Intraokularlinse nach einem der Ansprüche 1 bis 5, **dadurch gekennzeichnet, daß** der ringförmige Linsenbereich (3) eine Breite von etwa 0,8 mm bis 0,9 mm, insbesondere 0,835 mm aufweist.

7. Intraokularlinse nach einem der Ansprüche 1 bis 6, **dadurch gekennzeichnet, daß** der zentrale Linsenbereich (2) einen Durchmesser von etwa 4 mm aufweist.

8. Intraokularlinse nach einem der Ansprüche 1 bis 7, **dadurch gekennzeichnet, daß** der äußere Linsenrand (4) einen etwa halbkreisförmigen Querschnitt hat.

9. Intraokularlinse nach einem der Ansprüche 1 bis 8, **dadurch gekennzeichnet, daß** der zentrale Linsenbereich (2) eine glatte Oberfläche aufweist.

10. Intraokularlinse nach einem der Ansprüche 1 bis 9, **dadurch gekennzeichnet, daß** zur Bildung einer bivokalen Linse am optischen Linsenteil zusätzliche diffraktive Zonen (7) vorgesehen sind.

11. Intraokularlinse nach Anspruch 10, **dadurch gekennzeichnet, daß** die zusätzlichen diffraktiven Zonen (7) am zentralen, den refraktiven Anteil (2) bildenden zentralen Linsenbereich, vorgesehen sind.

12. Intraokularlinse nach einem der Ansprüche 1 bis 11, **dadurch gekennzeichnet, daß** der Weglängenunterschied zwischen den benachbarten diffraktiven Zonen (7) ein Bruchteil der Designwellenlänge ist.

4

13. Intraokularlinse nach einem der Ansprüche 1 bis 12, **dadurch gekennzeichnet, daß** der Weglängenunterschied zwischen den benachbarten diffraktiven Zonen (7) 0,4 oder 0,6 der Designwellenlänge beträgt.

14. Intraokularlinse nach einem der Ansprüche 1 bis 13, **dadurch gekennzeichnet, daß** die Designwellenlänge im grünen Spektralbereich des sichtbaren Lichtes liegt.

**Claims**

1. An intraocular lens comprising an optical lens portion which has a central lens region (2) and at least one further annular lens region (3) surrounding said central lens region (2), wherein the central lens region (2) and the at least one annular lens region (3) form a common focus, and wherein the annular lens region (3) has concentric annular zones in which the difference in the length of the beam path between adjacent zones is an integral multiple of $n \geq 2$ of the design wavelength, **characterised in that** the optical lens portion has an aspherical curvature configuration in the meridian section and that the annular region (3) with the concentric zones having the different beam path lengths is arranged in the lens portion in which the aspherical curvature configuration has its effect.

2. An intraocular lens according to claim 1 **characterised in that** the difference in beam path length is adjusted by the refractive index or the material and/or the geometry of the respective zone.

3. An intraocular lens according to claim 1 or claim 2 **characterised in that** the annular zones are of a sawtooth-like configuration.

4. An intraocular lens according to one of claims 1 to 3 **characterised in that** the annular zones are provided on the front and/or rear side of the lens body (1).

5. An intraocular lens according to one of claims 1 to 4 **characterised in that** a refractive component (2) is formed in the central lens region (2).

6. An intraocular lens according to one of claims 1 to 5 **characterised in that** the annular lens region (3) is of a width of from about 0.8 mm to 0.9 mm, in particular 0.835 mm.

7. An intraocular lens according to one of claims 1 to 6 **characterised in that** the central lens region (2) is of a diameter of about 4 mm.

8. An intraocular lens according to one of claims 1 to 7 **characterised in that** the outer lens edge (4) is of an approximately semicircular cross-section.

9. An intraocular lens according to one of claims 1 to 8 **characterised in that** the central lens region (2) has a smooth surface.

10. An intraocular lens according to one of claims 1 to 9 **characterised in that** additional diffractive zones (7) are provided for forming a bifocal lens on the optical lens portion.

11. An intraocular lens according to claim 10 **characterised in that** the additional diffractive zones (7) are provided at the central lens region forming the refractive component (2).

12. An intraocular lens according to one of claims 1 to 11 **characterised in that** the difference in beam path length between the adjacent diffractive zones (7) is a fraction of the design wavelength.

13. An intraocular lens according to one of claims 1 to 12 **characterised in that** the difference in beam path length between the adjacent diffractive zones (7) is 0.4 or 0.6 of the design wavelength.

14. An intraocular lens according to one of claims 1 to 13 **characterised in that** the design wavelength is in the green spectral range of visible light.

**Revendications**

1. Lentille intraoculaire présentant une partie de lentille optique qui comporte une région de lentille centrale (2) et, entourant celle-ci, au moins une autre région de lentille de forme annulaire (3), la région de lentille centrale (2) et la au moins une région de lentille de forme annulaire (3) formant un foyer commun, et la région de lentille de forme annulaire (3) comportant des zones de forme annulaire concentriques dans lesquelles la différence de trajet de la trajectoire des rayons entre des zones adjacentes est un multiple entier par $n \geq 2$ de la longueur d'ondes nominale,
   **caractérisée en ce que** la partie de lentille optique dans la section méridienne a un profil de courbure asphérique et que la région de forme annulaire (3) comportant les zones concentriques présentant les différents trajets est disposée dans la partie de lentille dans laquelle se manifeste l'effet du profil de courbure asphérique.

2. Lentille intraoculaire selon la revendication 1, **caractérisée en ce que** la différence de trajet est réglée par l'indice de réfraction ou le matériau et/ou la géométrie de la zone respective.

3. Lentille intraoculaire selon la revendication 1 ou 2, **caractérisée en ce que** les zones de forme annulaire sont réalisées à la manière de dents de scie.

4. Lentille intraoculaire selon l'une des revendications 1 à 3, **caractérisée en ce que** les zones de forme annulaire sont prévues sur la face avant et/ou la face arrière du corps de lentille (1).

5. Lentille intraoculaire selon l'une des revendications 1 à 4, **caractérisée en ce que** dans la région de lentille centrale (2) est formée une partie réfractive (2).

6. Lentille intraoculaire selon l'une des revendications 1 à 5, **caractérisée en ce que** la région de lentille de forme annulaire (3) présente une largeur d'environ 0,8 mm à 0,9 mm, en particulier de 0,835 mm.

7. Lentille intraoculaire selon l'une des revendications 1 à 6, **caractérisée en ce que** la région de lentille centrale (2) présente un diamètre d'environ 4 mm.

8. Lentille intraoculaire selon l'une des revendications 1 à 7, **caractérisée en ce que** le bord de lentille extérieur (4) a une section transversale sensiblement de forme semi-circulaire.

9. Lentille intraoculaire selon l'une des revendications 1 à 8, **caractérisée en ce que** la région de lentille centrale (2) présente une surface lisse.

10. Lentille intraoculaire selon l'une des revendications 1 à 9, **caractérisée en ce que** pour former une lentille bifocale des zones diffractives supplémentaires (7) sont prévues sur la partie de lentille optique.

11. Lentille intraoculaire selon la revendication 10, **caractérisée en ce que** les zones diffractives supplémentaires (7) sont prévues sur la zone de lentille centrale constituant la partie réfractive (2).

12. Lentille intraoculaire selon l'une des revendications 1 à 11, **caractérisée en ce que** la différence de trajet entre les zones diffractives (7) adjacentes est une fraction de la longueur d'ondes nominale.

13. Lentille intraoculaire selon l'une des revendications 1 à 12, **caractérisée en ce que** la différence de trajet entre les zones diffractives (7) adjacentes est de 0,4 ou 0,6 de la longueur d'ondes nominale.

14. Lentille intraoculaire selon l'une des revendications 1 à 13, **caractérisée en ce que** la différence de trajet se situe dans la plage verte du spectre de la lumière visible.

Fig. 1

Fig. 2